# EUROPÄISCHE PATENTSCHRIFT

(11) **EP 4 547 374 B1**
(45) Veröffentlichungstag und Bekanntmachung des Hinweises auf die Patenterteilung: **20.05.2026**
(21) Anmeldenummer: 23733926.2
(22) Anmeldetag: 16.06.2023
(51) Int. Cl.: B01D 53/62, B01D 53/81, B01J 20/04, B01J 20/16, B01J 20/28, G01N 33/00

(54) **ABSORPTIONSMITTEL ZUR QUANTITATIVEN ENTFERNUNG VON KOHLENDIOXID AUS EINEM GASSTROM SOWIE VERWENDUNG DESSELBEN**
ABSORBENT FOR THE QUANTITATIVE REMOVAL OF CARBON DIOXIDE FROM A GAS STREAM AND USE THEREOF
ABSORBANT POUR L'ÉLIMINATION QUANTITATIVE DU DIOXYDE DE CARBONE D'UN COURANT GAZEUX ET UTILISATION DE CELUI-CI

(30) Priorität: 30.06.2022 DE 102022116394
(43) Veröffentlichungstag der Anmeldung: 07.05.2025
(73) Patentinhaber: Elementar Analysensysteme GmbH, 63505 Langenselbold (DE)
(72) Erfinder: HARTWIG, Jan, 63755 Alzenau (DE); KRAUS, Sabine, 63755 Alzenau (DE)
(74) Vertreter: Keil & Schaafhausen Patentanwälte PartGmbB
(86) Internationale Anmeldenummer: PCT/EP2023/066272
(87) Internationale Veröffentlichungsnummer: WO 2024/002724

(56) Entgegenhaltungen:
- WO-A1-2015/101857
- WO-A1-98/17385
- CN-A- 110 614 020
- CN-A- 113 750 728
- RU-C1- 2 104 774

## Beschreibung

Die Erfindung betrifft eine Mischung zum quantitativen Entfernen von Kohlenstoffdioxid aus einem Gasstrom.

In der Analytik von Gasströmen ist es notwendig, Kohlenstoffdioxid (CO₂) quantitativ zu entfernen, wobei der Begriff quantitativ im Sinne der Erfindung so zu verstehen ist, dass der Kohlenstoffdioxidgehalt auf einen Wert von weniger als 1 ppm abgesenkt wird. Dies betrifft insbesondere die Analyse von Gasströmen mittels Wärmeleitfähigkeitsdetektor, IR-Spektroskopie oder einem Massenspektrometer. Üblicherweise werden daher die der Analytik zuzuführende Gasströme aufgereinigt, wobei in der Regel ein Absorptionsmittel in einem Rohr vorgesehen ist und der Gasstrom durch dieses Rohr geleitet wird.

Bisher war grundsätzlich die Verwendung von Natriumhydroxid (NaOH) als Absorptionsmittel bekannt, welche gemäß folgender Reaktionsgleichung abläuft:

2 NaOH + CO₂ → Na₂CO₃ + H₂O

Natriumhydroxid weist dabei eine ausreichende Reaktivität gegenüber Kohlenstoffdioxid aus, um tatsächlich eine quantitative Entfernung sicherzustellen. Nachteilig in der Analytik ist jedoch das dabei freiwerdende Wasser, welches jedoch für korrekte Messungen in der nachgeschalteten Analytik entfernt werden muss.

Allerdings kann das Natriumhydroxid nicht in Reinform verwendet werden, da es zur Bildung von Natriumcarbonat (NaCO₃) käme. Dieses Natriumcarbonat würde als Feststoffagglomerate formen und dadurch das Strömungsprofil des Gasstroms nachteilig verändern oder im schlimmsten Fall zu einer völligen Blockierung führen.

Ein weiterer Nachteil liegt bei der Verwendung in der Analytik von Gasströmen darin, dass sich das Natriumcarbonat primär an der Oberfläche des Bulks bildet, währen Natriumhydroxid im Inneren des Materials verbleibt. Beim Spülen mit Wasser löst sich dann sowohl das Carbonat als auch das Hydroxid und es entsteht eine stark basische und daher gefährliche Lösung.

In der Vergangenheit wurde daher Natriumhydroxid auf ein Trägermaterial, vorzugsweise einem Silicatträger, aufgebracht. Zumeist wurden auf diesem Trägermaterial zudem Quarzglassplitter, vorzugsweise im Verhältnis 1:1 zu Natriumhydroxid aufgebracht, um die Bildung von größeren Natriumcarbonatagglomeraten auch mechanisch zu unterbinden. Die Herstellung eines solchen Materials ist jedoch aufwendig. Zudem können nicht in allen Anwendungsmöglichkeiten Verblockungen mit den oben beschriebenen Nachteilen zuverlässig vollständig vermieden werden.

Aus dem Stand der Technik ist zudem die Verwendung von sogenanntem Natronkalk, einer Mischung aus Natriumhydroxid und Calciumhydroxid bekannt. Neben der Kohlenstoffdioxidentfernung in der Elementaranalyse wird die deshalb auch als Atemkalk bekannte Mischung in Kreislauftauchgeräten zur Bindung des ausgeatmeten Kohlenstoffdioxids verwendet.

Das enthaltene Calciumhydroxid (Ca(OH)₂) besitzt allein keine ausreichende Reaktivität, um Kohlenstoffdioxid in der benötigten Menge aus dem Gasstrom zu entfernen und ist daher als Absorptionsmittel für eine quantitative Entfernung von Kohlenstoffdioxid nicht geeignet. In der Mischung aus Natrium- und Calciumhydroxid reagiert in einer ersten Reaktion in bekannter Weise Natriumhydroxid auf der Oberfläche mit Kohlenstoffdioxid. In einer zweiten Reaktion reagiert dann das aus der Reaktion von Natriumhydroxid, mit Kohlenstoffdioxid entstandene Natriumcarbonat mit dem Calciumhydroxid ab. Aus dieser Reaktion entsteht Calciumcarbonat. Gleichzeitig wird das Natriumhydroxid regeneriert, wodurch die Kapazität des Absorptionsmittels stark gesteigert wird. Die Bruttoreaktionsgleichungen lauten dabei wie folgt:

2 NaOH + CO₂ → Na₂CO₃ + H₂O (schnell)

Na₂CO_{3 +} Ca(OH)₂ → 2 CaCO₃ + 2 NaOH

Diese Reaktionen sind jedoch stark wasserabhängig, was teilweise damit erklärt werden kann, dass nur in Anwesenheit von Wasser die chemischen Gleichgewichtsreaktionen eintreten, die zur Bildung von Carbonat- und Hydrogencarbonationen führen, was notwendige Zwischenstufen in den beiden Reaktionen sind.

Zu analysierende Gasströme sind jedoch zumeist bereits so vorgereinigt, dass sie nicht wassergesättigt sind und somit beim Durchströmen des Materials diesen Wasserüberschuss austragen würden. Dies ist jedoch aus einer Reihe von Gründen nachteilig: Gelangt der Wasser enthaltene Strom tatsächlich in den Detektor, würde er dessen Signal verfälschen. Alternativ müsste der Gasstrom nach der CO₂-Absorption getrocknet werden, was jedoch einen hohen Verbrauch an Trocknungsmittel mit sich bringen würde. Zudem würde der Austrag von Wasser aus dem System die ablaufenden Gleichgewichtsreaktionen negativ beeinflussen, so dass die zweite Reaktion mit Calciumhydroxid kaum mehr ablaufen könnte und sich binnen kürzester Zeit die Situation vergleichbar mit der Verwendung von reinem Natriumhydroxid darstellen würde.

Aus der CN 113 750 728 A ist die Verwendung einer Mischung aus NaOH und Ca(OH)₂ zur Entfernung von Kohlenstoffdioxid nach einer katalytischen Oxidation bekannt. Zusätzlich zu dem Natronkalk wird Wasser und ein Indikator zugeführt. Optional können auch weitere Adsorptionsmittel wie Molekularsieben (5A und 13X), oder ein CO₂-Adsorptionsmittel (bestehend aus NaOH, Perlit und Indikator) sowie zusätzliche Trockenmitteln zugefügt werden. Die Gewichtsanteile an der Gesamtmischung sind nicht offenbart.

Es ist daher Aufgabe der Erfindung, ein einfach und unbedenklich herstellbares Material zum quantitativen Entfernen von Kohlenstoffdioxid aus einem Gasstrom für eine nachgeschaltete Gasanalytik, insbesondere eine Elementaranalyse, bereitzustellen. Dies inkludiert auch, dass die Zusammensetzung des Gasstroms, insbesondere hinsichtlich des Wassergehalts, nicht so verändert werden darf, dass das nachgeschaltete Trockenmittel schnell verbraucht und/oder das Signal des Detektors verfälschen werden würde.

Diese Aufgabe wird mit einem Material gemäß Anspruch 1 gelöst.

Ein solches Material umfasst eine Mischung aus Natriumhydroxid, Calciumhydroxid und wenigstens einem Trocknungsmittel umfasst. Durch die zusätzliche Verwendung eines Trocknungsmittels ist sichergestellt, dass der zu analysierende Gasstrom keine größeren Mengen Wasser aus der Absorption des Kohlenstoffdioxids austrägt. Es ist allerdings zu beachten, dass die meisten Trocknungsmittel nicht pH-neutral sind. Als Beispiel für ein klassische Trockenmittel soll hier Sicapent^{®} als typischer saurer Vertreter genannt werden, welches daher selbst mit Natrium- oder Calciumhydroxid reagieren würden und daher als Mischungskomponente ungeeignet ist.

Zudem ist an das Trocknungsmittel die Anforderung zu stellen, dass das Trocknungsmittel das Wasser nicht so dauerhaft entfernen soll, dass es nicht mehr für das Reaktionsgleichgewicht zur Verfügung steht. Überraschenderweise wurde festgestellt, dass die Verwendung von Molekularsieb, auch Molsieb genannt, diesen Anforderungen gerecht wird.

Molekularsieb reagiert nicht mit Natriumhydroxid oder Calciumhydroxid. Gleichzeitig hält es das Wasser zuverlässig in der erfindungsgemäßen Mischung und beeinflusst so die Elementaranalytik oder nachgeschaltete Stufen wie eine Trocknung nicht zusätzlich. In gleichem Zug entfernt das Molekularsieb das Wasser jedoch nicht so vollständig aus der Mischung, dass die Regenerierung des Natriumhydroxids durch das Calciumhydroxid unterbunden wäre, da es innerhalb der Poren noch immer gleichgewichtswirksam vorhanden ist. Durch die simultane Erfüllung aller drei Anforderungen durch das verwendete Trocknungsmittel ist die Verwendung von Natriumhydroxid und Calciumhydroxid für die quantitative Entfernung von Kohlenstoffdioxid möglich.

Der positive Effekt der indirekten Bereitstellung des Wassers im Molekularsieb ist dabei so groß, dass die Standzeit des erfindungsgemäßen Materials höher ist als bei einem - die negativen Effekte von ausgetragenem Wasser außer Acht lassenden - Einsatz einer identischen Menge von Natrium- und Calciumhydroxid ohne die Verwendung eines Trocknungsmittels.

Der Begriff Molekularsieb ist im Sinne der Erfindung als funktionelle Bezeichnung für natürliche und synthetische Zeolithe sowie andere Stoffe zu verstehen, die eine hohe Adsorptionskapazität für Gase, Dämpfe und gelöste Stoffe mit bestimmten Molekülgrößen haben. Insbesondere bezieht sich die Erfindung auf natürliche und synthetische Zeolithe. Derartige Molekularsiebe weisen eine vergleichbare große innere Oberfläche, bevorzugt im Bereich von 500-760 m²/g auf und haben einen sehr homogenen Porendurchmesser, die in der Größenordnung der Durchmesser von Molekülen liegen. Zu Klassifizierung der Molekularesiebe wird in der Regel der in Angström angegebene Porendurchmesser verwendet.

Für die Mischung können Molsiebe zwischen 3 und 5 Å, insbesondere verschiedene 3 Å als auch 4 Å Molsiebe verwendet werden, wobei dies bedeutet, dass bevorzugt wenigstens 70 Gew.-%, vorzugsweise wenigstens 90 Gew.-% diesen Porendurchmesser aufweisen. 3 Å Molsiebe sind dabei besonders bevorzugt, da sie während der Messung im geringerem Maße Gas aufnehmen und wieder abgeben und so eine Verfälschung von Messergebnissen zuverlässig verhindern. Gleichzeitig halten sie Wasser zuverlässig zurück, dass keine zusätzliche Belastung einer ggf. nachgeschalteten Trocknung festzustellen ist.

Bevorzugt ist aufgrund des bereits beschriebenen Einflusses von Wasser auf die ablaufenden Reaktionen die eingesetzte Mischung aus Natriumhydroxid, Calciumhydroxid und einem Trocknungsmittel zusätzlich bereits von Beginn an Wasser umfasst und somit im Betrieb der Wassergehalt höher ist als derjenige, der aufgrund der Reaktion des Natriumhydroxids mit dem Kohlenstoffdioxid entsteht.

Erfindungsgemäß besteht die Mischung 0,5 bis 5 Gew.-% Natriumhydroxid, 20 bis 70 Gew.-% Calciumhydroxid und 25 bis 79,5 Gew. -% Trocknungsmittel, vorzugsweise 1 bis 3 Gew.-% Natriumhydroxid, 39 bis 59 Gew.-% Calciumhydroxid und 38 bis 62 Gew. -% Trocknungsmittel, besonders bevorzugt 1,5 bis 3 Gew.-% Natriumhydroxid, 39 bis 59 Gew.-% Calciumhydroxid und 38 bis 59,5 Gew.-% Trockenmitte wobei zusätzlich eine Vorsättigung mit Wasser erfolgt, so dass 1 bis 3 Gew.-% Natriumhydroxid, 33 bis 55 Gew.-% Calciumhydroxid, 3 bis 10 Gew.-% Wasser und 32 bis 63 Gew.-% Trocknungsmittel, bevorzugt 1,5 bis 3.0 Gew-% Natriumhydroxid, 35 bis 50 Gew.-% Calciumhydroxid, 4 bis 10 Gew.-% Wasser und 37 bis 59,5 Gew.-% Trockenmittel umfasst. Hierbei wird durch die verhältnismäßig geringe Menge an Natriumhydroxid die lokale Bildung von Natriumcarbonat und die damit verbundenen Blockierungen des Gasstroms zuverlässig vermieden. Gleichzeitig gewährt die Menge an Calciumhydroxid eine lange Standdauer des Absorptionsmittels. Die Menge des Trocknungsmittels und ggf. die Vorsättigung mit Wasser ist so abgestimmt, dass ein Wasserdurchbruch zuverlässig vermieden wird und gleichzeitig die Umsetzung des Calciumhydroxids nicht durch die vorhandene Wassermenge limitiert ist.

Es hat sich auch als günstig herausgestellt, wenn die Mischung zu wenigstens 90 Gew.-%, vorzugsweise 95 Gew.-% aus Natriumhydroxid, Calciumhydroxid, wenigstens einem Trocknungsmittel und Wasser besteht, so dass weitere Komponenten nicht die Reaktion beeinflussen.

In einer bevorzugten Ausgestaltung enthält das Material jedoch einen Indikator für Kohlenstoffdioxid und/oder einen Indikator für Wasser. Dies hat den Vorteil, dass beim Einsatz des Materials, beispielsweise dem Einfüllen einer Schüttung eventuelle Lagerfehler, welche zu einer Erschöpfung der Absorptionskapazitäten bereits vor dem Einsatz geführt haben, sichtbar sind. Gleichermaßen kann das Material auch in einem Gefäß mit zumindest teilweise durchsichtiger Ummantelung verwendet werden, so dass das erreichte Ende der Standzeit auch im Begriff visuell detektiert werden kann.

In einer besonders einfachen Ausgestaltung handelt es sich um einen pH-Indikator, welcher bei einer Erschöpfung des vorhandenen Calciumhydroxids und die daher nicht mehr ablaufende zweite Bruttoreaktion die dadurch bedingte pH-Änderung anzeigt.

Das Trocknungsmittel kann in jeder beliebigen Form eingesetzt werden, wobei sich für ein gleichmäßiges Strömungsprofil des Gasstroms eine granulare Form, insbesondere eine kugel- oder stäbchenförmige Ausgestaltung als besonders günstig herausgestellt hat

Die Erfindung umfasst ferner auch eine Vorrichtung zur Absorption von Kohlenstoffdioxid. Eine solche Vorrichtung weist ein gasdichtes Gehäuse mit einem Gaseinlass und einen Gasauslass auf. Das durch dieses Gehäuse definierte Volumen ist wenigstens teilweise mit dem erfindungsgemäßen Material nach einem der Ansprüche 1 bis 4 gefüllt ist. Eine besonders einfache Ausgestaltung einer solchen Vorrichtung ist ein befüllter Rohrreaktor.

Die Vorrichtung kann aus jedem Material gefertigt sein, welches sich gegenüber dem zu analysierenden Gasstrom inert verhält und gasdicht ist. Denkbar sind vor allem Metalle, Glas und Kunststoff. Glas und teilweise auch Kunststoff hat den Vorteil, dass es durchsichtig ist, Kunststoff bietet sich zudem aufgrund seiner Bruchfestigkeit und teilweise auch geringen Herstellungskosten besonders an.

In diesem Zusammenhang ist es auch möglich, diese Vorrichtung segmentweise mit einer Reihe von Materialien zu füllen, beispielsweise in Strömungsrichtung zunächst das erfindungsgemäße Material und nachgeschaltet ein Trocknungsmittel vorzusehen.

Schließlich richtet sich die Erfindung auch auf die Verwendung des erfindungsgemäßen Materials nach einem der Ansprüche 1 bis 5 und/oder einer erfindungsgemäßen Vorrichtung nach Anspruch 5 zur quantitativen Entfernung von Kohlenstoffdioxid in einem Gasanalytiksystem, insbesondere in der Elementaranalyse und/oder der IRMS. Wie beschrieben eignet sich das erfindungsgemäße Material hier in besonderer Weise, da neben einer quantitativen Entfernung auch sichergestellt sein muss, dass der von Kohlenstoffdioxid befreite Gasstrom nicht durch andere Komponenten wie beispielsweise Wasser verändert oder seine Strömungsverhalten verändert wurde.

Weitere Ausgestaltungsformen der Erfindung ergeben sich aus dem Beispiel sowie den Figuren mit ihrer zugehörigen Beschreibung. Dabei soll jedes Merkmal für sich und in beliebiger Kombination als offenbart gelten. Die Abbildungen sind teilweise leicht vereinfacht und schematisch.

Es zeigen:
- Fig. 1:: eine schematische Ausgestaltung einer erfindungsgemäßen Vorrichtung und
- Fig. 2:: schematisch den Einsatz einer erfindungsgemäßen Vorrichtung in einem Gasanalysesystem

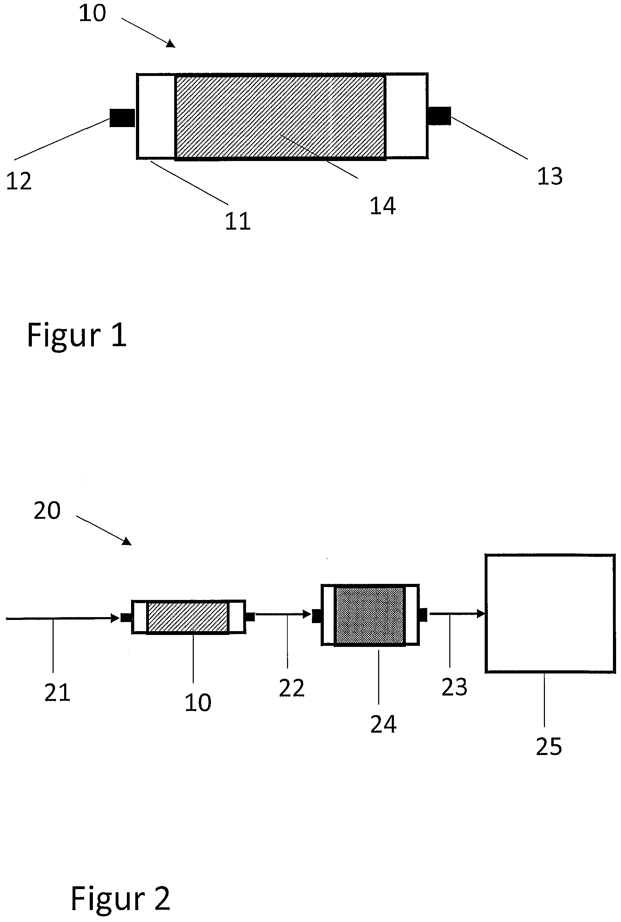

Figur 1 zeigt die erfindungsgemäße Vorrichtung 10, welche als, vorzugsweise zylinderförmiges, Gehäuse 11 mit einem Gaseinlass 12 und einem Gasauslass 13 ausgestattet ist. Im Inneren findet sich eine Füllung 14 mit dem erfindungsgemäßen Material, welches so eingebracht ist, dass der durch den Gaseinlass 12 einströmende Gasstrom in jedem Fall das Material passieren muss, bevor es über den Gasauslass 13 entweichen kann.

Figur 2 zeigt stark vereinfach ein Gasanalysesystem 20, vorzugsweise ein Elementaranalysesystem, in dem das erfindungsgemäße Material zur quantitativen Entfernung von Kohlenstoffdioxid eingesetzt wird. Über die Leitungen 21, 22 und 23 wird der Gasstrom zunächst über die erfindungsgemäße Vorrichtung 10 und dann eine Trocknungsvorrichtung 24 geführt, bevor er im Detektor 25 analysiert wird.

Alternativ zur graphischen Darstellung können auch die Funktionen der Vorrichtungen 10 und 24, also Kohlenstoffdioxidentfernung und Trocknung in einer Vorrichtung derart angeordnet sein, dass diese Vorrichtung zwei Segmente aufweist, in die ein entsprechendes Material eingefüllt ist. Insbesondere kann es sich dabei um ein Rohr handeln, welches in einem ersten Teil mit der erfindungsgemäßen Mischung und in einem zweiten, in Bezug auf den durchströmenden Gasstrom nachgeschalteten Teil mit einem Trocknungsmittel gefüllt ist.

Zudem kann eine nicht dargestellte Vortrocknung noch vor der Entfernung von Kohlenstoffdioxid vorgesehen sein.

### Beispiel

Das nachfolgende Beispiel zeigt, wie viele Messungen mit welcher Art von Absorptionsmittel für die quantitative Entfernung von Kohlenstoffdioxid durchgeführt werden können, bevor im Detektor ein von dem dann nicht mehr vollständig absorbierten Kohlenstoffdioxid und/oder von der nachgeschalteten Trockenvorrichtung nicht mehr vollständig entferntem Wasser stammendes Signal aufgezeichnet wird. Die angegebenen Werte sind Mittelwerte über jeweils 5 Standzeituntersuchungen.

| **Absorptionsmaterial** | **Anzahl Messungen** |
|---|---|
| Natriumhydroxid auf Trägermaterial | 300 |
| Natriumhydroxid/Calciumhydroxid (Strecke S1) mit anschließender Trocknung über eine zu durchströmende Strecke S2 (S1=S2) | 30 |
| Natriumhydroxid/Calciumhydroxid (Strecke S1) mit anschließender Trocknung über eine zu durchströmende Strecke S2, wobei S2=2*S1 ist | 60 |
| Erfindungsgemäße Mischung | 500 |

Es zeigt sich dabei, dass erfindungsgemäße Material allen anderen Absorptionsmaterialien hinsichtlich der Standzeit überlegen ist. Zudem kann die anschließende Trocknungsstrecke und damit verbunden auftretende Druckverluste bei der Verwendung der erfindungsmäßen Mischung vergleichsweise kurz ausgestaltet sein.

### Bezugszeichenliste

- 10: Vorrichtung
- 11: Gehäuse
- 12: Gaseinlass
- 13: Gasauslass
- 14: Füllung mit erfindungsgemäßem Material
- 20: Gasanalysesystem
- 21 - 23: Leitung
- 24: Trocknungsvorrichtung
- 25: Detektor

## Patentansprüche

1. Material zum quantitativen Entfernen von Kohlendioxid aus einem Gasstrom, wobei das Material eine Mischung aus Natriumhydroxid, Calciumhydroxid und wenigstens einem Trocknungsmittel enthält und dass das Trocknungsmittel ein Molekularsieb ist, **dadurch gekennzeichnet, dass** die Mischung 1 bis 3 Gew.-% Natriumhydroxid, 33 bis 55 Gew.-% Calciumhydroxid, 3 bis 10 Gew.-% Wasser und 32 bis 63 Gew.-% Trocknungsmittel umfasst.

2. Material zum quantitativen Entfernen von Kohlendioxid aus einem Gasstrom nach Anspruch 1, **dadurch gekennzeichnet, dass** das Molekularsieb zu wenigstens 70 Gew.-% eine Porenweite von 3 bis 5 Å aufweist.

3. Material zum quantitativen Entfernen von Kohlendioxid aus einem Gasstrom nach einem der vorhergehenden Ansprüche, **dadurch gekennzeichnet, dass** die Mischung zu wenigstens 90 Gew.-% aus Natriumhydroxid, Calciumhydroxid, wenigstens einem Trocknungsmittel und Wasser besteht.

4. Material zum quantitativen Entfernen von Kohlendioxid aus einem Gasstrom nach einem der vorhergehenden Ansprüche, **dadurch gekennzeichnet, dass** das Material einen Indikator für Kohlenstoffdioxid und/oder einen Indikator für Wasser enthält.

5. Vorrichtung (10) zur Absorption von Kohlenstoffdioxid mit einem gasdichten Gehäuse (11), welches einen Gaseinlass (12) und einen Gasauslass (13) aufweist und dessen Volumen wenigstens teilweise mit einem Material nach einem der Ansprüche 1 bis 4 gefüllt ist.

6. Verwendung eines Material nach einem der Ansprüche 1 bis 4 und/oder einer Vorrichtung nach Anspruch 5 zur quantitativen Entfernung von Kohlenstoffdioxid in einem einem Analysensystem, das mittels Gasanalyse arbeitet (20).

## Claims

1. Material for the quantitative removal of carbon dioxide from a gas stream, the material containing a mixture of sodium hydroxide, calcium hydroxide and at least one drying agent, and the drying agent being a molecular sieve, **characterized in that** the mixture comprises 1 to 3% by weight of sodium hydroxide, 33 to 55% by weight of calcium hydroxide, 3 to 10% by weight of water and 32 to 63% by weight of drying agent.

2. Material for the quantitative removal of carbon dioxide from a gas stream according to claim 1, **characterized in that** at least 70% by weight of the molecular sieve has a pore size of 3 to 5 Å.

3. Material for the quantitative removal of carbon dioxide from a gas stream according to one of the preceding claims, **characterized in that** the mixture consists of at least 90% by weight of sodium hydroxide, calcium hydroxide, at least one drying agent and water.

4. Material for the quantitative removal of carbon dioxide from a gas stream according to one of the preceding claims, **characterized in that** the material contains an indicator for carbon dioxide and/or an indicator for water.

5. Device (10) for absorbing carbon dioxide, having a gas-tight housing (11) which has a gas inlet (12) and a gas outlet (13) and whose volume is at least partially filled with a material according to one of claims 1 to 4.

6. Use of a material according to any one of claims 1 to 4 and/or a device according to claim 5 for the quantitative removal of carbon dioxide in an analytical system which operates by means of gas analysis (20).

## Revendications

1. Matériau pour l'élimination quantitative de dioxyde de carbone d'un courant gazeux, sachant que le matériau contient un mélange d'hydroxyde de sodium, d'hydroxyde de calcium et au moins un agent déshydratant et que l'agent déshydratant est un tamis moléculaire, **caractérisé en ce que** le mélange comprend 1 à 3 %/poids d'hydroxyde de sodium, 33 à 55 %/poids d'hydroxyde de calcium, 3 à 10 %/poids d'eau et 32 à 63 %/poids d'agent déshydratant.

2. Matériau pour l'élimination quantitative de dioxyde de carbone d'un courant gazeux selon la revendication 1, **caractérisé en ce que** le tamis moléculaire comporte pour au moins 70 %/poids une taille de pores de 3 à 5 A.

3. Matériau pour l'élimination quantitative de dioxyde de carbone d'un courant gazeux selon l'une quelconque des revendications précédentes, **caractérisé en ce que** le mélange pour au moins 90 %/poids est composé d'hydroxyde de sodium, d'hydroxyde de calcium, d'au moins un agent déshydratant et d'eau.

4. Matériau pour l'élimination quantitative de dioxyde de carbone d'un courant gazeux selon l'une quelconque des revendications précédentes, **caractérisé en ce que** le matériau contient un indicateur pour le dioxyde de carbone et/ou un indicateur pour l'eau.

5. Dispositif (10) pour l'absorption de dioxyde de carbone avec un boîtier étanche aux gaz (11), lequel comporte une entrée de gaz (12) et une sortie de gaz (13) et dont le volume de celui-ci est rempli au moins en partie avec un matériau selon l'une quelconque des revendications 1 à 4.

6. Utilisation d'un matériau selon l'une quelconque des revendications 1 à 4, et/ou d'un dispositif selon la revendication 5 pour l'élimination quantitative de dioxyde de carbone dans un système d'analyse, qui fonctionne au moyen de l'analyse de gaz (20).
